# EUROPEAN PATENT SPECIFICATION

(11) **EP 1 814 517 B1**
(45) Date of publication and mention of the grant of the patent: **11.08.2010**
(21) Application number: 05808069.8
(22) Date of filing: 19.10.2005
(51) Int. Cl.: A61K 9/08, A61K 31/19, A61K 31/216, A61K 47/00, C07C 229/42, C07C 57/38

(54) **NONAQUEOUS LIQUID PARENTERAL ACECLOFENAC FORMULATION**
NICHTWÄSSRIGE FLÜSSIGE PARENTERALE ACECLOFENAC-FORMULIERUNG
FORMULATION PARENTERALE D'ACECLOFENAC LIQUIDE NON AQUEUX

(30) Priority: 22.11.2004 IN DE23322004
(43) Date of publication of application: 08.08.2007
(73) Proprietor: Venus Remedies Limited, Chandigarh 160019 (IN)
(72) Inventor: CHAUDHARY, Manu, Panchkula 134 113, Haryana (IN)
(74) Representative: Meissner, Bolte & Partner
(86) International application number: PCT/IN2005/000339
(87) International publication number: WO 2006/054315

(56) References cited:
- EP-A- 1 231 209
- EP-A- 1 591 441
- US-A1- 2004 180 961
- ALONSO M J ET AL: "Aceclofenac-loaded poly(epsilon-caprolactone) nanocapsules. Effect of coadjuvants on morphometrical properties and drug entrapment" BOLLETTINO CHIMICO FARMACEUTICO, SOCIETA EDITORIALE FARMACEUTICA, MILANO, IT, vol. 139, no. 3, 2000, pages 114-119, XP002976061 ISSN: 0006-6648

## Description

### FIELD OF INVENTION

The present invention generally relates to nonaqueous liquid parenterally deliverable pharmaceutical formulation and more particularly to nonaqueous liquid parenteral Aceclofenac formulation comprising the selective NSAID Aceclofenac and still more particularly to pharmaceutically acceptable salts of Aceclofenac. The invention also relates to a process of preparing Aceclofenac formulation, the therapeutic dosage form and storage of dose, and the method of treating a subject having a condition or a disorder wherein treatment with NSAID is indicated.

### BACKGROUND OF INVENTION

The non-steroidal anti-inflammatory drug (NSAID) has a wide range and it is available both in non-prescription and prescription mode. The major groups of NSAID are as follows:
a. Salicyclic acid group: aspirin (acetyl salicyclic acid), Choline magnesium trisalicylate, diflunisal and salsalate.
b. Propionic acid group: fenoprofen, flurbiprofen, ibuprofen, ketoprofen, naproxen, and oxaprozin.
c. Acetic and acid group: diclofenac, aceclofenac, indomethacin, sulindac, and tolmetin.
d. Enolic acid group: meloxicam and piroxicam.
e. Fenamic acid group: meclofenamate and mefenamic acid.
f. Napthylalkenone group: nabumetone.
g. Pyarnocarboxylic acid group: etodalac.
h. Pyrrole group: Keterolac.
i. COX-2 inhibitor group: Celecoxib, valdecoxib and rofecoxib.

Some of different NSAIDs are available in one or more forms like tablets, injections, and dry powder for injection form. The present invention relates to "ready to use injection of aceclofenac."

The disadvantages of other NSAIDs as compared to the present invention are given below.
1) Dry powder injectable NSAID needs to be lyophilized and then reconstituted before use. The process is costly and cumbersome.
2) Injection form is more advantageous over oral form as serum concentration of oral NSAID is lesser because of intestinal metabolism of the drug.
3) Serum concentration of oral NSAID is unpredictable.
4) The frequency of administration of oral NSAID is higher.
5) Diclofenac has more side-effects than Aceclofenac.
6) NSAID's injections such as indometacin have lesser tolerability than Aceclofenac.
7) Ketoprofen is less stable than Aceclofenac.

Parenteral drug formulations have become a very important component in the arsenal of available drug delivery options, particularly for drugs having analgesic effect. Parenteral routes of administration, including subcutaneous, intramuscular and intravenous injection, offer numerous benefits over oral delivery in particular situations, for a wide variety of drugs. For example, parenteral administration of a drug typically results in attainment of a therapeutically effective blood serum concentration of the drug in a shorter time than is achievable by oral administration. This is especially true of intravenous injection, whereby the drug is placed directly in the bloodstream. Parenteral administration can also result in more predictable blood serum concentrations of a drug, because losses in the gastrointestinal tract due to metabolism, binding to food and other causes are eliminated. For similar reasons, parenteral administration often permits dose reduction. Parenteral administration is generally the preferred method of drug delivery in emergency situations, and is also useful in treating subjects who are uncooperative, unconscious, or otherwise unable or unwilling to accept oral medication, and where a faster relief is required in lesser time, specially in acute and chronic painful condition.

If a parenteral drug formulation is to be prepared, it is preferable from patient convenience and safety standpoints that such a formulation be a ready-to-use formulation, i.e. one that does not require dilution or mixing immediately prior to use (as opposed to a reconstitutable formulation). Ready-to-use and dilutable liquid parenteral formulations can also be advantageous from a manufacturing standpoint by avoiding expensive lyophilization and/or other similar manufacturing steps.

Aceclofenac is a phenyl acetic acid derivative which inhibits interleukin1β-induced prostaglandin E2 production but has poor COX-I inhibitor effect in oral form. This formulation converts it to the Diclofenac, the COX-I and COX-II inhibitor, following administration to a subject, itself shows enhanced inhibitory activity against both COX-1 and COX-2.

Because Diclofenac has very poor gastrointestinal (GI) tolerability ,it is not particularly well suited for formulation. By contrast, because of the better GI tolerability of aceclofenac and lesser side effects than diclofenac, Aceclofenac has been proposed by the inventor for liquid formulation.

Unfortunately, attempts to formulate Aceclofenac as a ready-to-use solution for injection have heretofore been complicated by the fact that Aceclofenac , when in solution and especially in presence of certain excipients, is unstable, and undergoes degradation which may precipitate out making injection unsuitable for use.

One potential solution to this problem is to provide a dry reconstitutable Aceclofenac formulation which is mixed with a liquid vehicle just prior to administration but this too is not stable for longer period. However, in many situations it is particularly advantageous to provide a liquid formulation, more especially a ready-to-use formulation, as indicated above.

If a liquid parenterally deliverable formulation of Aceclofenac or a pharmaceutically acceptable salt thereof, particularly such a formulation that is ready-to-use, that is storage stable at room temperature could be prepared, a significant advance in treatment of COX-2 mediated conditions and disorders would result. This Would be especially true for such conditions and disorders characterized by or accompanied by pain, particularly where rapid onset of pain relief is desired (as, for example, in migraine and other forms of acute and/or severe pain).

US Patent application publication no. 20040180961 of Lee, Beom-Jin, et al discuss the composition and preparation method for bioavailable oral Aceclofenac dosage forms. The composition contains water-insoluble Aceclofenac, a polymeric base and a surfactant. It is stated that the oral preparation according to this invention has excellent solubility in gastrointestinal tract, thereby improving dissolution rate and thus bioavailability, as well as rapid dispersion and dissolution properties in gastrointestinal tract. In addition, when being orally administered in an amount much smaller than the conventional preparation, the oral preparation is therapeutically effective, thus minimizing gastrointestinal disorder.

G Pasero et al, in their paper "A multi-centre, double-blind comparative study of the efficacy and safety of aceclofenac and diclofenac in the treatment of rheumatoid arthritis" of Current Medical Research Opinion, (1995), 13, 305, discuss the effectiveness of aceclofenac in detail, as given below:

Rheumatoid arthritis (RA) is a chronic inflammatory disease of the joint space which involves synovial proliferation and cartilage destruction. Non-steroidal anti-inflammatory drugs (NSAIDs) are considered to be the first-line symptomatic treatment for RA. Since most patients require chronic NSAID therapy it is important for an NSAID to be well tolerated in addition to being effective. Indeed, the threat of serious gastro-intestinal complications is a major concern of long-term NSAID therapy.

Aceclofenac is a new NSAID of the phenylacetic acid class of NSAIDs (2-[(2,6-dichlorophenyl)amino]phenylacetoxyacetic acid), which has been shown to exhibit good analgesic, antipyretic and anti-inflammatory efficacy in several animal models of acute and chronic inflammation. Although maintaining a similar pharmacodynamic profile to that of indomethacin and diclofenac, aceclofenac has shown better gastric tolerance when compared to other NSAIDs. In rodents, the acute gastric ulcerogenic activity of aceclofenac was found to be 2-, 4- and 7-fold less than naproxen, diclofenac or indomethacin, respectively. Indeed the therapeutic index for aceclofenac was reported to be four times greater than that of diclofenac, a well-established NSAID in clinical use.

Short-term clinical studies have demonstrated the efficacy of aceclofenac in pain relief following dental extraction and episiotomy and in the chronic treatment of rheumatoid arthritis (RA) and osteoarthritis. Furthermore, two studies have demonstrated that aceclofenac is at least as effective as ketoprofen and better tolerated, in terms of fewer drop-outs, in the long-term treatment of RA. In some comparative studies in joint diseases, there was a tendency for aceclofenac to be better tolerated than diclofenac or ketoprofen, with fewer patients being withdrawn from treatment due to gastric intolerance.

This long-term comparative study of aceclofenac and diclofenac in RA has confirmed that the therapeutic efficacy of aceclofenac is comparable to that of diclofenac, a well-established NSAID, in the treatment of rheumatoid arthritis. During the study, both drugs improved the degree of pain, as indicated by the VAS and the Ritchie Index. The fact that, at baseline, both these variables were significantly greater in the aceclofenac than in the diclofenac group suggests that the RA disease may have been more severe in the aceclofenac group and that the efficacy of this drug may have been somewhat greater, since there was no difference between the groups after 6 months. This possibility is supported by the fact that aceclofenac tended to be more effective than diclofenac in improving the Ritchie Index and grip strength. In a previous 3 month study in RA, aceclofenac was found to be significantly more effective than ketoprofen, both with regard to the Ritchie Index and morning stiffness.

The main disadvantage of long-term therapy with NSAIDs is the risk of gastro-intestinal disturbances. NSAIDs carry a greater risk of inducing upper gastro-intestinal bleeding than simple analgesics, though the risk is dependent on the dose and is highest in patients who have previously suffered bleeding episodes. Ibuprofen and diclofenac have recently been shown to be the NSAIDs, of those most frequently used, which have the lowest risk of causing upper gastro-intestinal bleeding. Any NSAID which, therefore, has comparable or better gastro-intestinal tolerability than these two drugs is likely to be well accepted for long-term therapy of RA. Preclinical studies demonstrated that aceclofenac not only has a superior therapeutic activity but also a more favourable ulcerogenic index than diclofenac, phenylbutazone or indomethacin. Furthermore, results of previous comparative clinical trials with ketoprofen showed that aceclofenac, while being similar in terms of therapeutic efficacy, demonstrates a better safety and tolerability profile. In the present study, aceclofenac was also well tolerated by patients, the incidence of gastro-intestinal intolerance tending to be lower in the aceclofenac group (13%) than in the diclofenac group (17%). These findings not only confirm the results of previous studies in RA demonstrating the good tolerability of the drug, they also support earlier findings in healthy volunteers showing that aceclofenac tended to be more tolerable than diclofenac in terms of gastro-intestinal blood loss.

Gonzalez-Alvaro et al, in their paper " Aceclofenac, a new nonsteroidal anti-inflammatory drug, decreases the expression and function of some adhesion molecules on human neutrophils" of J Rheumatol. 1996 Apr;23(4):723-9, discuss the effects of Aceclofenac as given below:

Aceclofenac induced a dramatic decrease of L-selectin expression, whereas a moderate and slight decrement of CD43 and ICAM-3 expression was also observed. In contrast, the expression of other adhesion molecules by neutrophils was unaffected (CD11a, CD31, CD44) or slightly increased (CD11b). Cell adhesion assays, performed under nonstatic conditions, revealed that aceclofenac significantly diminished the L-selectin dependent neutrophil adhesion to endothelial cells. Neutrophil aggregation induced with anti-CD43 Mab was also significantly inhibited by aceclofenac. CONCLUSION. Aceclofenac had a faster and more potent effect than the other NSAID studied, mainly on the expression of cell adhesion molecules. This new NSAID efficiently interferes with neutrophil adhesion to endothelium and this effect may represent an additional relevant mechanism in its anti-inflammatory activity.

Grau M. et al, in their paper " Pharmacology of the potent new non-steroidal anti-inflammatory agent aceclofenac" of Arzneimittelforschung. 1991 Dec; 41 (12):1265-76 state about Aceclofenac as under:

Aceclofenac (2-[(2,6-dichlorophenyl) amine]phenylacetoxyacetic acid; CAS 89796-99-6) is a new orally effective non-steroidal anti-inflammatory agent of the phenylacetic acid group which showed remarkable anti-inflammatory, analgesic, and antipyretic properties. Hence, aceclofenac possesses a potent inhibitory activity in several models of acute and chronic inflammation in rodents, and resembles indometacin and diclofenac in its pharmacodynamic profile, being superior to naproxen and phenylbutazone. In addition, aceclofenac was found to be highly active against sodium urate-induced synovitis in dogs and adjuvant-induced polyarthritis in rats, both prophylactically and therapeutically. The analgesic effect of aceclofenac on the pain elicited by chemical and mechanical stimuli was nearly equal to or slightly better than that of indometacin and diclofenac. Fever induced by brewer's yeast injection in rats was also markedly suppressed by aceclofenac. In contrast, the acute gastric ulcerogenic activity of aceclofenac was about 2, 4 and 7-fold lesser than that of naproxen, diclofenac, or indometacin, respectively. As a consequence of its high anti-inflammatory activity and lower potential for gastric damage aceclofenac exhibited the most favourable therapeutic ratio in comparison with indometacin, diclofenac, naproxen, and phenylbutazone. These data indicate that aceclofenac could be a potent anti-inflammatory and analgesic agent with a wide margin of safety in clinical practice.

Grau M. et al, in their paper, "The pharmacological profile of aceclofenac, a new nonsteroidal anti-inflammatory and analgesic drug" of Agents Actions Supplement 1991, state about Aceclofenac as follows:
Aceclofenac is a new phenylacetic acid derivative provided with marked anti-inflammatory, antiarthritic, analgesic and antipyretic activities in animal experimental models. While maintaining its potency Aceclofenac demonstrates better gastric tolerance and consequently offers greater potential security than other highly active agents such as Indomethacin and Diclofenac.

Schattenkirchner et al, in their paper "A double-blind, multicentre, randomised clinical trial comparing the efficacy and tolerability of aceclofenac with diclofenac resinate in patients with acute low back pain" of Clinical Rheumatology, 2003 May, discuss Aceclofenac as under:

In conclusion, non-inferiority of the analgesic efficacy of aceclofenac compared with diclofenac resinate was demonstrated in patients with localised, uncomplicated acute lumbosacral pain. For the reduction in pain levels from baseline there was also evidence for superiority of aceclofenac compared with diclofenac resinate in terms of statistical significance, although this difference was not considered clinically relevant. The results also showed a trend towards a better safety and tolerability profile of aceclofenac over diclofenac resinate from a clinical point of view.

Hence in view of the above discussions there is a perceived need for development of Aceclofenac in stable injection form and a method of preparing the same injection and also a method of treating a subject having a condition or disorder wherein a very urgent treatment with a NSAID is indicated, this very urgent treatment being given by administering Aceclofenac in an injection form.

### Objects and advantages of the invention

Accordingly, the objects and advantages of the present invention are described as below:
An object of the present invention is to provide Aceclofenac in ready-to-use injection form.

Other object of the present invention is to provide a formulation which is convenient to use and stable for much longer time as compared to dry powder aceclofenac injection.

Another object of the present invention is to provide Aceclofenac injection so that effective therapeutic concentration in serum is obtained in shorter time and lesser dose than oral NSAID.

Yet another object of the present invention is to provide Aceclofenac injection so that serum concentration is more accurately predictable than oral NSAID, because intestinal metabolism of the drug is not involved.

Still another object of the present invention is to provide Aceclofenac injection when giving oral dose to patient is not possible.

Further object of the present invention is to provide Aceclofenac injection which has better safety and tolerability profile as compared to Diclofenac from the clinical point of view.

Yet further object of the present invention is to provide Aceclofenac injection which has safer, faster and more potent effects than some of other NSAIDs.

Still further object of the present invention is to provide Aceclofenac injection, which has lesser side-effects than Diclofenac, the closest NSAID injection.

Another object of the present invention is to provide NSAID liquid parenteral formulation that does not require dilution or mixing or reconstitution immediately prior to use.

Yet another object of the present invention is to provide a NSAID ready-to-use liquid perenteral formulation which is storage-stable at room temperature.

Further object of the present invention is to develop a process for preparing liquid parenteral NSAID formulation which will avoid use of expensive lyophilization and/or other similar manufacturing steps.

Still further object of the present invention is to provide a method of treating a subject having a condition or disorder wherein a very urgent treatment with NSAID is indicated.

### DESCRIPTION OF INVENTION

According to its most preferred embodiment, the present invention provides a nonaqueous liquid parenteral Aceclofenac formulation, capable of pharmaceutical application. The present invention comprises Aceclofenac component in a form of non-water-soluble Aceclofenac salt in a solubilized and/or dissolved form in a solvent liquid, wherein the solvent liquid comprises;
a. a nonaqueous solubilizer component effective to stabilize Aceclofenac and also Diclofenac that forms by conversion of Aceclofenac thereto, the nonaqueous solubilized component being substantially inert with respect to this conversion; and
b. an Aceclofenac salt stabilizer component effective to inhibit precipitation of Aceclofenac free acid.

The nonaqueous solubilizer component and the Aceclofenac salt stabilizer component are same or alternatively, different.

When the Aceclofenac formulation is stored in a closed sealed airtight container, which is maintained at 30 °C for a period of 180 days, the Aceclofenac constitutes at least about 98 % by weight of Aceclofenac free acid, of the total amount of formulation.

The Aceclofenac formulation alternatively comprises additional one or more salicylic acid derivative(s) and nonaqueous solubilizer components, the additional nonaqueous stabilizer components being substantially inert.

The Aceclofenac salt of the present invention comprises a phenyl acetic acid derivative with anti-inflammatory analgestic properties similar to those of Diclofenac.

In the Aceclofenac formulation of this invention, the Aceclofenac expressed as free acid is present in an amount in the range from about 1 mg/ml to about 400 mg/ml, preferably from about 1 mg/ml to about 300 mg/ml and more preferably from about 10 mg/ml to about 50 mg/ml of the formulation.

The Aceclofenac salt stabilizer component comprises an oxygen limiting means for limiting the effective exposure of the Aceclofenac formulation to oxygen, wherein the oxygen limiting means comprises one or more antioxidants which are selected from the group consisting of butylated hydroxyanisole, propyl gallate and butylated hydroxytoluene. The total amount of antioxidants is in the range from about 0.001% to about 5% by weight of the solvent liquid. The oxygen limiting means further comprises an inert gas limited microatmosphere in contact with the Aceclofenac formulation.

The salicylic acid derivative component of the present invention helps to stabilize the injection and improve efficiency. The component is in the range of 1% to 500% of aceclofenac, preferably, 50% to 250% of aceclofenac and more preferably 75% to 150% of aceclofenac.

The nonaqueous solubilizer component for this Aceclofenac formulation is selected from the group consisting of propylene glycol, polyethylene glycol and optional ethanol, or alternatively, any two of these or all three of these.

When propylene glycol is used in this invention, the concentration of propylene glycol is more than 7% by weight of the solvent liquid formed by mixture of nonaqueous solubilizer component and Aceclofenac salt stabilizer component. Alternatively the concentration is in the range from about 7% to about 70%.

When polyethylene glycol is used, the concentration thereof is from about 35% to about 95%, preferably above 30% by weight of the solvent liquid. The polyethylene glycol has an average molecular weight ranging from about 200 to about 1000 and preferably from about 300 to about 800.

When optional ethanol is used, the concentration thereof is in the range from about 1% to about 30% by weight of the solvent liquid.

When Benzyl alchohol is used as preservative, the concentration thereof is in the range from about 0.1 % to about 4%.

Compositions of the invention can be provided at a Aceclofenac concentration suitable for parenteral delivery without mixing and/or dilution immediately prior to administration (i.e., "ready-to-use"), yet such compositions are surprisingly stable upon storage at room temperature and at refrigerated temperatures. Compositions of the invention, whether ready-to-use or requiring dilution prior to administration, can be prepared by processes that avoid the need for an expensive and time consuming lyophilization step. Other features of this invention will be in part apparent and in part pointed out hereinafter.

According to its another embodiment, the present invention provides a process for preparing a nonaqueous liquid parenteral Aceclofenac formulation for pharmaceutical application, which process comprises of the steps of;
(A) Combining in specific order and mixing:
   a. Aceclofenac component in a form of non-water-soluble Aceclofenac salt,
   b. A non aqueous solubilizer component effective to stabilize aceclofenac and diclofenac that forms by conversion of aceclofenac thereto, the non-aqueous solubilizer component being substantially inert with respect to this conversion,
   c. An aceclofenac salt stabilizer component such as salicylic acid derivatives and the optional Aceclofenac salt stabilizer component comprising oxygen limiting factors,
   d. An optional ethanol component,
   e. A Benzyl alcohol component.
(B) Solubilizing and/or dissolving aceclofenac salt in the solvent liquid formed by mixture of the components at (b), (c), (d) and (e) above, whereby, upon the storage of the non-aqueous liquid parenteral aceclofenac formulation in a close container maintained at 30 °C for a period of 180 days, the aceclofenac constitutes at least about 98 % by weight of the total amount of the aceclofenac formulation, expressed as Aceclofenac free acid, in the formulation.

Alternatively, in this process, the aceclofenac salt stabilizer component of step (c) above comprises one or more stabilizing agents selected from the group consisting of pH controlling means, antioxidants, propylene glycol and polyethylene glycol. At least one of the non aqueous solubilizer component and the aceclofenac salt stabilizer component is propylene glycol. The concentration of propylene glycol is more than 7 % by weight of the aceclofenac formulation.

The different components used in this process invention satisfy the same condition as described under description of the most preferred embodiment of this invention.

According to yet another embodiment of the present invention is provided a pharmaceutically effective dosage of the non aqueous liquid parenteral aceclofenac formulation, wherein the aceclofenac salt is present at a suitable concentration for parenteral administration without further dilution. The required amount of dosage is provided in a sealed airtight container which is selected from the group consisting of a vial, and ampoule, a syringe, a packet, a pouch and an auto-injector. The interior space of the sealed airtight container comprises a fill volume occupied by the aceclofenac formulation and a headspace volume occupied by an inert-gas-limited micro-atmosphere, which micro-atmosphere comprises essentially of one or more inert gases selected from the group consisting of noble gases and nitrogen, such that the ratio of the fill volume to headspace volume is not less than 1:1.

When the nitrogen is used, the headspace volume has a nitrogen volume of not more than about 5% so as to replace oxygen in the headspace volume.

The aceclofenac salt is present in the sealed container in an amount corresponding to single unit dose when the sealed airtight container is preferably a glass ampoule. Alternatively, the amount of the Aceclofenac salt in the sealed airtight container is corresponding to 1 to 20 unit doses.

The Aceclofenac salt is preferably Aceclofenac sodium, which is filled asceptically under inert gas blanket.

According to its a further embodiment, the present invention provides a method of treating a subject having a condition or disorder wherein a treatment with NSAID is indicated, which method comprises parenterally administering a therapeutically effective amount of the nonaqueous liquid parenteral Aceclofenac formulation.

### Therapeutic use of Compositions of the Invention:

Compositions of the invention are useful in treatment and prevention of a very wide range of disorders mediated by-COX-2, including but not restricted to disorders characterized by inflammation, pain and/or fever. Such compositions are especially useful as anti-inflammatory agents, such as in treatment of arthritis, with the additional benefit of having significantly less harmful side effects than compositions of conventional NSAIDs. In particular, compositions of the invention have reduced potential for gastrointestinal toxicity and gastrointestinal irritation, including upper gastrointestinal ulceration and bleeding, by comparison with compositions of conventional NSAIDs.

Contemplated compositions are useful to treat a variety of arthritic disorders, including but not limited to rheumatoid arthritis, spondyloarthropathies particularly ankylating spondalosis, osteoarthritis, systemic lupus erythematosus and juvenile arthritis.

Such compositions are useful in treatment of pain and inflammation caused by asthma, bronchitis, menstrual cramps, preterm labor, tendonitis, bursitis, allergic neuritis, cytomegalovirus infection, apoptosis including HIV-induced apoptosis, lumbago, skin-related conditions such as psoriasis, eczema, acne, burns, dermatitis and ultraviolet radiation damage including sunburn, and postoperative inflammation including that following ophthalmic surgery such as cataract surgery or refractive surgery.

Such compositions are useful in treating pain and inflammation in such diseases as migraine headaches, periarteritis, thyroiditis, aplastic anemia, Hodgkin's disease, scleroderma, rheumatic fever, type 1 diabetes, neuromuscular junction disease including myasthenia gravis, white matter disease including multiple sclerosis, sarcoidosis, nephrotic syndrome, Behcet's syndrome, polymyositis, gingivitis, nephritis, hypersensitivity, swelling occurring after injury including brain edema, myocardial ischemia, and the like.

Such compositions are useful in treatment of pain and inflammation caused by ophthalmic disorders, including without limitation inflammatory disorders such as endophthalmitis, episcleritis, retinitis, iriditis, cyclitis, choroiditis, keratitis, conjunctivitis and blepharitis, inflammatory disorders of more than one part of the eye, e.g., retinochoroiditis, iridocyclitis, iridocyclochoroiditis (also known as uveitis), keratoconjunctivitis, blepharoconjunctivitis, etc.; other COX-2 mediated retinopathies; ocular photophobia; acute trauma of any tissue of the eye including postsurgical trauma, e.g., following cataract or corneal transplant surgery; postsurgical ocular inflammation; intraoperative miosis; ocular, for example retinal, neovascularization including that following injury or infection; macular degeneration; cystoid macular edema; retrolental fibroplasia; neovascular glaucoma; and ocular pain.

Such compositions are useful in treatment of pain caused by pulmonary inflammation, such as that associated with viral infections and cystic fibrosis, and in bone resorption such as that associated with osteoporosis.

Such compositions are useful for treatment of pain and inflammation caused by certain central nervous system disorders, such as cortical dementias including Alzheimer's disease, neurodegeneration, and central nervous system damage resulting from stroke, ischemia and trauma. The term "treatment" in the present context includes partial or total inhibition of dementias, including Alzheimer's disease, vascular dementia, multi-infarct dementia, pre-senile dementia, alcoholic dementia and senile dementia.

Such compositions are useful in treatment of pain and inflammation caused by allergic rhinitis, respiratory distress syndrome, endotoxin shock syndrome and liver disease.

Such compositions are useful in treatment of pain, including but not limited to postoperative pain, dental pain, muscular pain, and pain resulting from cancer. For example, such compositions are useful for relief of pain, fever and inflammation in a variety of conditions including rheumatic fever, influenza and other viral infections including common cold, low back and neck pain, dysmenorrhea, headache, toothache, sprains and strains, myositis, neuralgia, synovitis, arthritis, including rheumatoid arthritis, degenerative joint diseases (osteoarthritis), gout and ankylosing spondylitis, bursitis, bums, and trauma following surgical and dental procedures.

Such compositions are useful for treating and preventing inflammation-related cardiovascular disorders, including vascular diseases, coronary artery disease, aneurysm, vascular rejection, arteriosclerosis, atherosclerosis including cardiac transplant atherosclerosis, myocardial infarction, embolism, stroke, thrombosis including venous thrombosis, angina including unstable angina, coronary plaque inflammation, bacterial-induced inflammation including Chlamydia-induced inflammation, viral induced inflammation, and inflammation associated with surgical procedures such as vascular grafting including coronary artery bypass surgery, revascularization procedures including angioplasty, stent placement, endarterectomy, or other invasive procedures involving arteries, veins and capillaries.

Such compositions are useful in treatment of pain and inflammation caused by angiogenesis-related disorders in a subject, for example to inhibit tumor angiogenesis. Such compositions are useful in treatment of neoplasia, including metastasis; ophthalmological conditions such as corneal graft rejection, ocular neovascularization, retinal neovascularization including neovascularization following injury or infection, diabetic retinopathy, macular degeneration, retrolental fibroplasia and neovascular glaucoma; ulcerative diseases such as gastric ulcer; pathological, but non-malignant, conditions such as hemangiomas, including infantile hemangiomas, angiofibroma of the nasopharynx and avascular necrosis of bone; and disorders of the female- reproductive system such as endometriosis.

Such compositions are useful in prevention and treatment of pain and inflammation caused by benign and malignant tumors and neoplasia including cancer, such as colorectal cancer, brain cancer, bone cancer, epithelial cell-derived neoplasia (epithelial carcinoma) such as basal cell carcinoma, adenocarcinoma, gastrointestinal cancer such as lip cancer, mouth cancer, esophageal cancer, small bowel cancer, stomach cancer, colon cancer, liver cancer, bladder cancer, pancreas cancer, ovary cancer, cervical cancer, lung cancer, breast cancer, skin cancer such as squamous cell and basal cell cancers, prostate cancer, renal cell carcinoma, and other known cancers that effect epithelial cells throughout the body. Neoplasias for which compositions of the invention are contemplated to be particularly useful are gastrointestinal cancer, Barrett's esophagus, liver cancer, bladder cancer, pancreatic cancer, ovarian cancer, prostate cancer, cervical cancer, lung cancer, breast cancer and skin cancer. Such compositions can also be used to treat fibrosis that occurs with radiation therapy. Such compositions can be used to treat subjects having adenomatous polyps, including those with familial adenomatous polyposis (FAP). Additionally, such compositions can be used to prevent polyps from forming in subjects at risk of FAP.

Such compositions inhibit prostanoid-induced smooth muscle contraction by inhibiting synthesis of contractile prostanoids and hence can be of use in treatment of pain caused by dysmenorrhea, premature labor, asthma and cosinophil-related disorders. They also can be of use for decreasing bone loss particularly in postmenopausal women (i.e., treatment of osteoporosis), and for treatment of pain caused by glaucoma.

Preferred uses for compositions of the present invention are for treatment of rheumatoid arthritis and osteoarthritis, for pain management generally (particularly post-oral surgery pain, post-general surgery pain, post-orthopedic surgery pain, and acute flares of osteoarthritis), for prevention and treatment of headache.

Besides being useful for human treatment, compositions of the invention are useful for veterinary treatment of companion animals, exotic animals, farm animals, and the like, particularly mammals. More particularly, compositions of the invention are useful for treatment of COX-2 mediated disorders in horses, dogs and cats. ,

The present invention is further directed to a therapeutic method of treating a condition or disorder where treatment with a COX-2 inhibitory drug is indicated, the method comprising parenteral administration of a composition of the invention to a subject in need thereof. The dosage regimen to prevent, give relief from, or ameliorate the condition or disorder preferably corresponds to once-a-day or twice-a-day treatment, but can be modified in accordance with a variety of factors. These include the type, age, weight, sex, diet and medical condition of the subject and the nature and severity of the disorder. Thus, the dosage regimen actually employed can vary widely and can therefore deviate from the preferred dosage regimens set forth herein.

Initial treatment can begin with a dose regimen as indicated herein. Treatment is generally continued as necessary over a period of several weeks to several months or years until the condition or disorder has been controlled or eliminated. Subjects undergoing treatment with a composition of the invention can be routinely monitored by any of the methods well known in the art to determine effectiveness of therapy.

Continuous analysis of data from such monitoring permits modification of the treatment regimen during therapy so that optimally effective doses are administered at any point in time, and so that the duration of treatment can be determined. In this way, the treatment regimen and dosing schedule can be rationally modified over the course of therapy so that the lowest amount of the composition exhibiting satisfactory effectiveness is administered, and so that administration is continued only for so long as is necessary to successfully treat the condition or disorder.

The term "parenteral administration" herein encompasses injection of a composition by means other than through the gastrointestinal tract such as into or through the skin of a subject, and includes intramuscular administration. Any known device useful for parenteral injection of drugs can be used to effect such administration.

The term "effective dose" herein means a dose that is deemed to be effective for a medical purpose (e.g. prophylactic or therapeutic) and will vary depending upon many factors. Such non-limiting factors include route and frequency of administration and medical purpose.

The term "unit dose" herein means an amount of Aceclofenac being suitable for delivery in a single administration event.

Thus according to an embodiment of the present invention, a method is provided for treatment of a COX-2 mediated disorder in a human subject comprising parenterally administering a composition as described herein to the subject at a Aceclofenac dosage equal to a therapeutically effective dosage of Diclofenac.

Preferably, the Aceclofenac salt is administered in a daily dosage amount of about 1 mg to about 300 mg. More preferred daily dosage amounts are about 10 mg to about 250 mg, more preferably about 30 mg to about 200 mg, and still more preferably about 50 mg to about 150 mg, for example about 100 mg or about 150 mg, aceclofenac.

The present compositions can be used in combination therapies with opioids and other analgesics, including narcotic analgesics, Mu receptor antagonists, Kappa receptor antagonists, non-narcotic (i.e. non-addictive) analgesics, monoamine uptake inhibitors, adenosine regulating agents, cannabinoid derivatives, Substance P antagonists, neurokiin-1 receptor antagonists with dose adjustments.

Preferred combination therapies comprise use of a composition of the invention with one or more compounds selected from acemetacin, .epsilon.-acetamidocaproic acid, acetaminophen, acetaminosalol, acetanilide, acetylsalicylsalicylic acid, S-adenosyhnethionine, alclofenac, alfentanil, allylprodine, alminoprofen, aloxiprin, alphaprodine, aluminum bis(acetylsalicylate), amfenac, amino chlorthenoxazin, 3-amino-4-hydroxybutyric acid, 2-amino-4-picoline, aminopropylon, aminopyrine, amixetrine, ammonium salicylate, ampiroxicam, amtolmetin guacil, anileridine, antipyrine, antipyrine salicylate, antrafenine, apazone, aspirin, balsalazide, bendazac, benorylate, benoxaprofen, benzpiperylon, benzydamine, benzylmnorphine, berberine, bermoprofen, bezitramide, alpha.-bisabolol, bromfenac, p-bromoacetanilide, 5-bromosalicylic acid acetate, bromosaligenin, bucetin, bucloxic acid, bucolome, bufexamac, bumadizon, buprenorphine, butacetin, butibufen, butorphanol, calcium acetylsalicylate, carbamazepine, carbiphene, carprofen, carsalam, chlorobutanol, chlorthenoxazin, choline salicylate, cinchophen, cinmetacin, ciramadol, clidanac, clometacin, clonitazene, clonixin, clopirac, clove, codeine, codeine methyl bromide, codeine phosphate, codeine sulfate, cropropamide, crotethamide, desomorphine, dexoxadrol, dextromoramide, dezocine, diampromide, diclofenac, difenamizole, difenpiramide, diflunisal, dihydrocodeine, dihydrocodeinone enol acetate, dihydromorphine, dihydroxyaluminum acetylsaiicylate, dimenoxadol, dimepheptanol, dimethylthiambutene, dioxaphetyl butyrate, dipipanone, dipyrocetyl, dipyrone, ditazol, droxicam, emorfazone, enfenamnic acid, epirizole, eptazocine, etanercept, etersalate, ethenzamide, ethoheptazine, ethoxazene, ethylmethylthiambutene, ethylmorphine, etodolac, etofenamate, etonitazene, eugenol, felbinac, fenbufen, fenclozic acid, fendosal, fenoprofen, fentanyl, fentiazac, fepradinol, feprazone, floctafenine, flufenamic acid, flunoxaprofen, fluoresone, flupirtine, fluproquazone, flurbiprofen, fosfosal, gentisic acid, glafenine, glucametacin, glycol salicylate, guaiazulene, hydrocodone, hydromorphone, hydroxypethidine, ibufenac, ibuprofen, ibuproxam, imidazole salicylate, indomethacin, indoprofen, infliximab, interleukin-10, isofezolac, isoladol, isomethadone, isonixin, isoxepac, isoxicam, ketobemidone, ketoprofen, ketorolac, p-lactophenetide, lefetamine, levorphanol, lexipafant, lofentanil, lonazolac, lomoxicam, loxoprofen, lysine acetylsalicylate, magnesium acetylsalicylate, meclofenamic acid, mefenamic acid, meperidine, meptazinol, mesalamine, metazocine, methadone, methotrimeprazine, metiazinic acid, metofoline, metopon, mofebutazone, mofezolac, morazone, morphine, morphine hydrochloride, morphine sulfate, morpholine salicylate, myrophine, nabumetone, nalbuphine, 1-naphthyl salicylate, naproxen, narceine, nefopam, nicomorphine, nifenazone, niflumic acid, nimesulide, 5'-nitro-2'-propoxyacetanilide, norlevorphanol, normethadone, normorphine, norpipanone, olsalazine, opium, oxaceprol, oxametacine, oxaprozim, oxycodone, oxymorphone, oxyphenbutazone, papaveretum, paranyline, parsalmide, pentazocine, perisoxal, phenacetin, phenadoxone, phenazocine, phenazopyridine hydrochloride, phenocoll, phenoperidine, phenopyrazone, phenyl acetylsalicylate, phenylbutazone, phenyl salicylate, phenyramidol, piketoprofen, piminodine, pipebuzone, piperylone, pirazolac, piritramide, piroxicam, pirprofen, pranoprofen, proglumetacin, proheptazine, promedol, propacetamol, propiram, propoxyphene, propyphenazone, proquazone, protizinic acid, ramifenazone, remifentanil, rimazolium metilsulfate, salacetamnide, salicin, salicylamide, salicylamide o-acetic acid, salicylsulfuric acid, salsalate, salverine, simetride, sodium salicylate, sufentanil, sulfasalazine, sulindac, superoxide dismutase, suprofen, suxibuzone, talniflumate, tenidap, tenoxicam, terofenamate, tetrandrine, thiazolinobutazone, tiaprofenic acid, tiaramide, tilidine, tinoridine, tolfenamic acid, tolmetin, tramadol, tropesin, viminol, xenbucin, ximoprofen, zaltoprofen, ziconotide and zomepirac (see The Merck Index, 13th Edition (2001), Therapeutic Category and Biological Activity Index, lists therein headed "Analgesic" , "Anti-inflammatory" and "Antipyretic").

## Claims

1. A nonaqueous liquid parenteral Aceclofenac formulation, capable of pharmaceutical application, comprising an Aceclofenac component in a form of a non-water-soluble Aceclofenac salt, in a solubilized and / or dissolved form in a solvent liquid wherein said solvent liquid comprises;
a) a nonaqueous solubilizer component effective to stabilize Aceclofenac and Diclofenac that forms by conversion of Aceclofenac thereto, said nonaqueous solubilizer component being substantially inert with respect to said conversion ;
and
b) an Aceclofenac salt stabilizer component effective to inhibit precipitation of Aceclofenac free acid.

2. The Aceclofenac formulation of Claim 1, wherein said formulation when stored in a closed sealed airtight container, which is maintained at 30 degree Celcius for a period of 180 days, Aceclofenac, expressed as Aceclofenac- free acid, constitutes at least about 98 % of the total amount of said formulation.

3. The Aceclofenac formulation of Claim 1, wherein said nonaqueous solubilizer component and said Aceclofenac salt stabilizer component are same or alternatively, different.

4. The Aceclofenac formulation of Claim 1, wherein said formulation further comprises one or more salicylic acid derivatives as stabilizer component and other optional Aceclofenac salt stabilizer component comprising oxygen limiting means.

5. The Aceclofenac formulation of Claim 1, wherein said formulation further comprises additional one or more nonaqueous solubilizer components, effective to stabilize said Aceclofenac and said Diclofenac that forms by conversion of said Aceclofenac thereto,
said additional nonaqueous solubilizer components being substantially inert with respect to said conversion.

6. The Aceclofenac formulation of Claim 1, wherein said Aceclofenac salt comprises a phenyl acetic acid derivative with anti-inflammatory analgesic properties similar to those of said Diclofenac

7. The Aceclofenac formulation of Claim 1, wherein said Aceclofenac expressed as free acid, is present in an amount ranging from about 1 mg/ml to about 400 mg/ml, preferably from about 1 mg/ml to 300 mg/ml and more preferably from 10 mg/ml to 50 mg/ml of said formulation.

8. The Aceclofenac formulation of any of Claims 1 and 5, wherein said nonaqueous solubilizer component is selected from the group consisting of polyethylene glycol and optional ethanol.

9. The Aceclofenac formulation of any of Claims 1 and 5 wherein said nonaqueous solubilizer component is selected from the group consisting of polyethylene glycol and optional ethanol.

10. The Aceclofenac formulation of any of Claims 1 and 5 wherein said nonaqueous solubilizer component is selected from the group consisting of propylene glycol and polyethylene glycol.

11. The Aceclofenac formulation of any of claims 1 and 5, wherein Benzyl alcohol is used as a preservative.

12. The Aceclofenac formulation of any of Claims 8 and 10, wherein concentration of said propylene glycol is more than 7 % , by weight of said solvent liquid of claim 1 which is formed by mixture of said nonaqueous solubilizer component and said Aceclofenac salt stabilizer component.

13. The Aceclofenac formulation of any of Claims 8 and 10, wherein concentration of said propylene glycol is within the range from about 7 % to about 70 % by weight of said solvent liquid of claim 1 which is formed by mixture of said nonaqueous solubilizer component and said Aceclofenac salt stabilizer component.

14. The Aceclofenac formulation of any of Claims 8, 9 and 10, wherein concentration of said polyethylene glycol is within range from about 35 % to about 95 %, preferably above 30 % by weight of said solvent liquid of Claim 1 , which is formed by mixture of said nonaqueous solubilizer component and said Aceclofenac salt stabilizer component, and wherein said polyethylene glycol has an average molecular weight within range from about 200 to about 1000 and preferably within range from about 300 to about 800.

15. The Aceclofenac formulation of Claim 11, wherein concentration of said Benzyl alcohol is in the range from about 0.1% to about 4% by weight of said solvent liquid of Claim 1.

16. The Aceclofenac formulation of Claim 4, wherein concentration of said salicylic acid derivatives is within range from about 1% to about 500%, preferably from about 50% to about 250% and more preferably from about 75% to about 150% by weight of said Aceclofenac salt of Claim 1.

17. The Aceclofenac formulation of any of Claims 8 and 9, wherein concentration of said optional ethanol is within range from about I % to about 30 % by weight of said solvent liquid of claim 1, which is formed by mixture of said nonaqueous solubilizer component and said Aceclofenac salt stabilizer component.

18. The Aceclofenac formulation of any of Claims 1 , 3 and 4, wherein said Aceclofenac salt stabilizer component comprises an oxygen limiting means for limiting the effective exposure of said Aceclofenac formulation to oxygen, wherein said means comprises one or more antioxidants which are selected from the group consisting of butylated hydroxyanisole , propyl gallate and butylated hydroxytoluence, the total amount of said antioxidant being within the range from about 0.001 % to about 5 % byweight of said solvent liquid, and wherein said means further comprises an inert gas limited atmosphere in contact with said Aceclofenac formulation.

19. The Aceclofenac formulation of Claim 1, wherein a pharmaceutically effective dosage thereof includes said Aceclofenac salt at a suitable concentration for parenteral administration without further dilution.

20. The Aceclofenac formulation of Claim 19 wherein required amount of said effective dosage is provided in a sealed airtight container which is selected from the group consisting of a vial, an ampoule, a syringe, a packet, a pouch and an auto - injector.

21. The Aceclofenac formulation of Claim 20, wherein interior space of said sealed airtight container comprises a fill volume occupied by said Aceclofenac formulation and a headspace volume occupied by and inert - gas - limited microatmosphere, which microatmosphere comprises essentially of one or more inert gases selected from the group consisting of noble gases and nitrogen such that the ratio of said fill volume to said headspace volume is not less than 1 :1.

22. The Aceclofenac formulation of claim 21 , wherein said inert gas of nitrogen in said headspace volume has a nitrogen volume of not more than 5 %, so as to replace oxygen in said headspace volume.

23. The Aceclofenac formulation of Claim 19, wherein said Aceclofenac salt is present in an amount corresponding to single unit dose, wherein said sealed airtight container of any of Claims 20 and 21 is preferably a glass ampoule.

24. The Aceclofenac formulation of Claim 19 wherein said Aceclofenac salt is present in an amount corresponding to any of single 1 to 20 unit doses.

25. The Aceclofenac formulation of Claim 19 wherein said Aceclofenac salt is Aceclofenac Sodium, which is filled aseptically under inert gas blanket.

26. A process for preparing a nonaqueous liquid parenteral Aceclofenac formulation for pharmaceutical application, which process comprises the steps of:
(A) combining in specific order and mixing :
(a) an Aceclofenac component in a form of non - water - soluble Aceclofenac salt,
(b) a nonaqueous solubilizer component effective to stabilize Aceclofenac and Diclofenac that forms by conversion of Aceclofenac thereto, said nonaqueous solubilizer component being substantially inert with respect to said conversion,
(c) an Aceclofenac salt stabilizer component such as salicylic acid derivatives and optional Aceclofenac salt stabilizer component comprising oxygen limiting factors,
(d) an optional ethanol component, and
(e) a Benzyl alcohol component,
(B) solubilizing and/or dissolving Aceclafenac salt in the solvent liquid formed by mixture of the components of (b), (c), (d), and (e) above of step (A), whereby, upon storage of said nonaqueous liquid parenteral Aceclofenac formulation in a closed container maintained at 30 degree Celcius for a period of 180 days, said Aceclofenac constitutes at least about 98 % by weight of said Aceclofenac formulation, expressed as said Aceclofenac free acid in said formulation.

27. The process of Claim 26, wherein said Aceclofenac salt stabilizer component of step (c) of step (A), comprises one or more stabilizing agents selected from the group consisting of pH controlling means, antioxidants, propylene glycol and polyethylene glycol.

28. The process of Claim 26, wherein at least one of said non aqueous solubilizer component and said Aceclofenac salt stabilizer component is polyethylene glycol.

29. The process of Claim 27, wherein concentration of said propylene glycol is more than 7 % by weight of said formulation.

30. Use of a nonaqueous liquid Aceclofenac formulation according to Claim 1, for the preparation of a parenterally administrable therapeutically effective amount for treating a subject having a condition or disorder wherein a treatment with NSAID is indicated.

31. Use of a nonaqueous liquid Aceclofenac formulation according to claim 30, wherein said disorder is COX-2 mediated and wherein said parenterally administering therapeutically effective amount corresponds to a daily dosage of nonaqueous liquid Aceclofenac of about 1 mg per day to about 300 mg per day, preferably of about 10 mg per day to about 250 mg per day, more preferably of about 30 mg per day to about 200 mg per day, still more preferably of about 50 mg per day to about 150 mg per day.

## Patentansprüche

1. Nichtwässrige, flüssige, parenterale Aceclofenac-Formulierung, geeignet für pharmazeutische Anwendung, einen Aceclofenac-Inhaltsstoff in Form eines wasserunlöslichen Salzes von Aceclofenac in einer aufgeschlossenen und/oder gelösten Form in Lösungsmittelflüssigkeit umfassend, wobei die Lösungsmittelflüssigkeit umfasst:
a) einen nichtwässrigen Aufschlussmittelinhaltsstoff, der wirksam ist, um Aceclofenac und Diclofenac zu stabilisieren, das sich **dadurch** bildet, dass Aceclofenac dazu umgesetzt wird, wobei der nichtwässrige Aufschlussmittelinhaltsstoff im Hinblick auf die Umsetzung im Wesentlichen inert ist; und
b) einen Aceclofenacsalz-Stabilisatorinhaltsstoff, der wirksam ist, um ein Präzipitieren von freier Säure von Aceclofenac zu verhindern.

2. Aceclofenac-Formulierung nach Anspruch 1, wobei in der Formulierung, wenn sie in einem geschlossenen, versiegelten, luftdichten Behälter gelagert wird, der für eine Dauer von 180 Tagen auf 30 Grad Celsius gehalten wird, Aceclofenac, als freie Säure von Aceclofenac ausgedrückt, mindestens ca. 98% der Gesamtmenge der Formulierung ausmacht.

3. Aceclofenac-Formulierung nach Anspruch 1, wobei der nichtwässrige Aufschlussmittelinhaltsstoff und der Aceclofenacsalz-Stabilisatorinhaltsstoff gleich oder alternativ unterschiedlich sind.

4. Aceclofenac-Formulierung nach Anspruch 1, wobei die Formulierung darüber hinaus ein oder mehrere Salicylsäurederivat/e als Stabilisatorinhaltsstoff und andere optionale Aceclofenacsalz-Stabilisatorinhaltsstoffe umfasst, die Sauerstoff einschränkende Mittel umfassen.

5. Aceclofenac-Formulierung nach Anspruch 1, wobei die Formulierung darüber hinaus einen oder mehrere zusätzliche/n nichtwässrige/n Aufschlussmittelinhaltsstoff/e umfasst, der/die wirksam ist/sind, um das Aceclofenac und das Diclofenac, das sich **dadurch** bildet, dass das Aceclofenac dazu umgesetzt wird, zu stabilisieren, wobei die zusätzlichen nichtwässrigen Aufschlussmittelinhaltsstoffe im Hinblick auf die Umsetzung im Wesentlichen inert sind.

6. Aceclofenac-Formulierung nach Anspruch 1, wobei das Aceclofenacsalz ein Phenylessigsäurederivat mit entzündungshemmenden analgetischen Eigenschaften ähnlich denjenigen des Diclofenacs umfasst.

7. Aceclofenac-Formulierung nach Anspruch 1, wobei das als freie Säure ausgedrückte Aceclofenac in einer Menge vorhanden ist, die von ca. 1 mg/ml bis ca. 400 mg/ml, vorzugsweise von ca. 1 mg/ml bis 300 mg/ml, und noch bevorzugter von 10 mg/ml bis 50 mg/ml der Formulierung reicht.

8. Aceclofenac-Formulierung nach einem der Ansprüche 1 und 5, wobei der nichtwässrige Aufschlussmittelinhaltsstoff aus der Gruppe ausgewählt ist, die aus Propylenglykol, Polyethylenglykol und optionalem Ethanol besteht.

9. Aceclofenac-Formulierung nach einem der Ansprüche 1 und 5, wobei der nichtwässrige Aufschlussmittelinhaltsstoff aus der Gruppe ausgewählt ist, die aus Polyethylenglykol und optionalem Ethanol besteht.

10. Aceclofenac-Formulierung nach einem der Ansprüche 1 und 5, wobei der nichtwässrige Aufschlussmittelinhaltsstoff aus der Gruppe ausgewählt ist, die aus Propylenglykol und Polyethylenglykol besteht.

11. Aceclofenac-Formulierung nach einem der Ansprüche 1 und 5, wobei Benzylalkohol als Konservierungsmittel verwendet wird.

12. Aceclofenac-Formulierung nach einem der Ansprüche 8 und 10, wobei eine Konzentration des Propylenglykols höher ist als 7 Gew.-% der Lösungsmittelflüssigkeit von Anspruch 1, die durch Mischen des nichtwässrigen Aufschlussmittelinhaltsstoffs und des Aceclofenacsalz-Stabilisatorinhaltsstoffs gebildet wird.

13. Aceclofenac-Formulierung nach einem der Ansprüche 8 und 10, wobei eine Konzentration des Propylenglykols im Bereich von ca. 7 Gew.-% bis ca. 70 Gew.-% der Lösungsmittelflüssigkeit von Anspruch 1 liegt, die durch Mischen des nichtwässrigen Aufschlussmittelinhaltsstoffs und des Aceclofenacsalz-Stabilisatorinhaltsstoffs gebildet wird.

14. Aceclofenac-Formulierung nach einem der Ansprüche 8, 9 und 10, wobei eine Konzentration des Polyethylenglykols im Bereich von ca. 35 Gew.-% bis ca. 90 Gew.-%, vorzugsweise über 30 Gew.-% der Lösungsmittelflüssigkeit von Anspruch 1 liegt, die durch Mischen des nichtwässrigen Aufschlussmittelinhaltsstoffs und des Aceclofenacsalz-Stabilisatorinhaltsstoffs gebildet wird, und wobei das Polyethylenglykol ein durchschnittliches Molekulargewicht im Bereich von ca. 200 bis ca. 1000, und vorzugsweise im Bereich von ca. 300 bis ca. 800 hat.

15. Aceclofenac-Formulierung nach Anspruch 11, wobei eine Konzentration des Benzylalkohols im Bereich von ca. 0.1 Gew.-% bis ca 4 Gew.-% der Lösungsmittelflüssigkeit von Anspruch 1 liegt.

16. Aceclofenac-Formulierung nach Anspruch 4, wobei eine Konzentration der Salicylsäurederivate im Bereich von ca. 1 Gew.-% bis ca. 500 Gew.-%, vorzugsweise von ca. 50 Gew.-% bis ca. 250 Gew.-%, und noch bevorzugter von ca. 75 Gew.-% bis ca. 150 Gew.-% des Aceclofenacsalzes von Anspruch 1 liegt.

17. Aceclofenac-Formulierung nach einem der Ansprüche 8 und 9, wobei eine Konzentration des optionalen Ethanols in einem Bereich von ca. 1 Gew.-% bis ca. 30 Gew.-% der Lösungsmittelflüssigkeit von Anspruch 1 liegt, die durch Mischen des nichtwässrigen Aufschlussmittelinhaltsstoffs und des Aceclofenacsalz-Stabilisatorinhaltsstoffs gebildet wird.

18. Aceclofenac-Formulierung nach einem der Ansprüche 1, 3 und 4, wobei der Aceclofenacsalz-Stabilisatorinhaltsstoff ein Sauerstoff einschränkendes Mittel umfasst, um die effektive Einwirkung von Sauerstoff auf die Aceclofenac-Formulierung einzuschränken, wobei das Mittel ein Antioxidans oder mehrere Antioxidantien umfasst, die aus der Gruppe ausgewählt sind, die aus Butylhydroxyanisol, Propylgallat und Butylhydroxytoluol besteht, wobei die Gesamtmenge des Antioxidans im Bereich von ca. 0,001 Gew.-% bis ca. 5 Gew.-% der Lösungsmittelflüssigkeit liegt, und wobei das Mittel darüber hinaus eine auf Inertgas beschränkte Atmosphäre umfasst, die mit der Aceclofenac-Formulierung in Kontakt ist.

19. Aceclofenac-Formulierung nach Anspruch 1, wobei eine pharmazeutisch wirksame Dosierung von dieser Aceclofenacsalz in einer zur parenteralen Verabreichung ohne weitere Verdünnung geeigneten Konzentration umfasst.

20. Aceclofenac-Formulierung nach Anspruch 19, wobei eine erforderliche Menge der wirksamen Dosierung in einem versiegelten, luftdichten Behälter bereitgestellt wird, der aus der Gruppe ausgewählt ist, die aus einem Fläschchen, einer Ampulle, einer Spritze, einer Packung, einem Beutel und einem Autoinjektor besteht.

21. Aceclofenac-Formulierung nach Anspruch 20, wobei ein Innenraum des versiegelten, luftdichten Behälters ein durch die Aceclofenac-Formulierung eingenommenes Füllvolumen und ein durch eine auf Inertgas beschränkte Mikroatmosphäre eingenommenes Kopfraumvolumen umfasst, welche Mikroatmosphäre sich im Wesentlichen aus einem oder mehreren Inertgas/en zusammensetzt, die aus der Gruppe ausgewählt sind, die aus Edelgasen und Stickstoff besteht, derart, dass das Verhältnis des Füllvolumens zum Kopfraumvolumen nicht weniger als 1 : 1 beträgt.

22. Aceclofenac-Formulierung nach Anspruch 21, wobei das Inertgas Stickstoff im Kopfraumvolumen ein Stickstoffvolumen von nicht mehr als 5 % hat, um Sauerstoff im Kopfraumvolumen zu ersetzen.

23. Aceclofenac-Formulierung nach Anspruch 19, wobei das Aceclofenacsalz in einer Menge vorhanden ist, die einer Einzeleinheitsdosis entspricht, wobei es sich bei dem versiegelten, luftdichten Behälter von einem der Ansprüche 20 und 21 vorzugsweise um eine Glasampulle handelt.

24. Aceclofenac-Formulierung nach Anspruch 19, wobei das Aceclofenacsalz in einer Menge vorhanden ist, die einer einzelnen Dosis mit 1 bis 20 Einheiten entspricht.

25. Aceclofenac-Formulierung nach Anspruch 19, wobei es sich bei dem Aceclofenacsalz um Aceclofenac-Natrium handelt, das unter einer Inertgashülle aseptisch abgefüllt ist.

26. Verfahren zur Zubereitung einer nichtwässrigen, flüssigen, parenteralen Aceclofenac-Formulierung zur pharmazeutischen Anwendung, das die folgenden Schritte umfasst:
(A) Kombinieren in einer festgelegten Reihenfolge und Mischen
- (a) eines Aceclofenac-Inhaltsstoffs in Form eines wasserunlöslichen Aceclofenacsalzes,
- (b) eines nichtwässrigen Aufschlussmittelinhaltsstoffs, der wirksam ist, um Aceclofenac und Diclofenac, das sich **dadurch** bildet, dass Aceclofenac dazu umgesetzt wird, zu stabilisieren, wobei der nichtwässrige Aufschlussmittelinhaltsstoff im Hinblick auf die Umsetzung im Wesentlichen inert ist,
- (c) einen Aceclofenacsalz-Stabilisatorinhaltsstoff wie etwa Salicylsäurederivate und einen optionalen Aceclofenacsalz-Stabilisatorinhaltsstoff, der Sauerstoff einschränkende Faktoren umfasst,
- (d) einen optionalen Ethanolinhaltsstoff, und
- (e) einen Benzylalkoholinhaltsstoff,
(B) Aufschließen und/oder Lösen des Aceclofenacsalzes in der Lösungsmittelflüssigkeit, die durch Mischen der vorstehenden Inhaltsstoffe (b), (c), (d) und (e) von Schritt (A) gebildet wurde, wodurch beim Lagern der nichtwässrigen, flüssigen, parenteralen Aceclofenac-Formulierung in einem geschlossenen Behälter, der für eine Dauer von 180 Tagen auf 30 Grad Celsius gehalten wird, das Aceclofenac, ausgedrückt als freie Aceclofenacsäure in der Formulierung, mindestens ca. 98 Gew.-% der Aceclofenac-Formulierung ausmacht.

27. Verfahren nach Anspruch 26, wobei der Aceclofenacsalz-Stabilisatorinhaltsstoff des Schritts (c) von Schritt (A) ein oder mehrere stabilisierende/s Mittel umfasst, das/die aus der Gruppe ausgewählt ist/sind, die aus ph-regelnden Mitteln, Antioxidantien, Propylenglykol und Polyethylenglykol besteht.

28. Verfahren nach Anspruch 26, wobei es sich bei dem nichtwässrigen Aufschlussmittelinhaltsstoff und/oder Aceclofenacsalz-Stabilisatorinhaltsstoff um Polyethylenglykol handelt.

29. Verfahren nach Anspruch 27, wobei die Konzentration des Propylenglykols mehr als 7 Gew.-% der Formulierung beträgt.

30. Verwendung einer nichtwässrigen, flüssigen Aceclofenac-Formulierung nach Anspruch 1, zur Zubereitung einer parenteral verabreichbaren, therapeutisch wirksamen Menge, um eine Person zu behandeln, die einen Zustand oder eine Erkrankung hat, bei dem bzw. der eine Behandlung mit NSAID indiziert ist.

31. Verwendung einer nichtwässrigen, flüssigen Aceclofenac-Formulierung nach Anspruch 30, wobei die Erkrankung durch COX-2 vermittelt ist, und wobei die parenteral verabreichbare, therapeutisch wirksame Menge einer täglichen Dosis des nichtwässrigen flüssigen Aceclofenacs von ca. 1 mg pro Tag bis ca. 300 mg pro Tag, vorzugsweise von ca. 10 mg pro Tag bis ca. 250 mg pro Tag, bevorzugter von ca. 30 mg pro Tag bis ca. 200 mg pro Tag, noch bevorzugter von ca. 50 mg pro Tag bis ca. 150 mg pro Tag entspricht.

## Revendications

1. Formulation parentérale d'acéclofénac liquide non aqueux, convenant à une application pharmaceutique, comprenant un composant acéclofénac sous forme de sel d'acéclofénac non soluble dans l'eau, sous forme solubilisée et/ou dissoute dans un liquide solvant, ledit liquide solvant comprenant :
a) un composant solubilisant non aqueux permettant de stabiliser l'acéclofénac et le diclofénac qui se forme par conversion d'acéclofénac, ledit composant solubilisant non aqueux étant sensiblement inerte par rapport à cette conversion ; et
b) un composant stabilisant de sel d'acéclofénac permettant d'inhiber la précipitation d'acide libre d'acéclofénac.

2. La formulation d'acéclofénac selon la revendication 1, où, dans ladite formulation, lorsqu'elle est stockée dans un récipient fermé, scellé, étanche à l'air qui est maintenu à 30 degrés Celsius pendant une durée de 180 jours, l'acéclofénac, exprimé en acide libre d'acéclofénac, constitue au moins environ 98 % de la quantité totale de la dite formulation.

3. La formulation d'acéclofénac selon la revendication 1, où ledit composant solubilisant non aqueux et ledit composant stabilisant de sel d'acéclofénac sont pareils ou, en variante, différents.

4. La formulation d'acéclofénac selon la revendication 1, où ladite formulation comprend en outre un ou plusieurs dérivés d'acide salicylique comme composant stabilisant et autre composant stabilisant de sel d'acéclofénac facultatif comprenant un moyen de limitation d'oxygène.

5. La formulation d'acéclofénac selon la revendication 1, où ladite formulation comprend en outre un ou plusieurs composants solubilisants non aqueux supplémentaires permettant de stabiliser ledit acéclofénac et ledit diclofénac qui se forme par conversion dudit acéclofénac, lesdits composants solubilisants non aqueux supplémentaires étant sensiblement inertes par rapport à ladite conversion.

6. La formulation d'acéclofénac selon la revendication 1, où ledit sel d'acéclofénac comprend un dérivé d'acide phénylacétique ayant des propriétés analgésiques anti-inflammatoires similaires à celles dudit diclofénac.

7. La formulation d'acéclofénac selon la revendication 1, où ledit acéclofénac, exprimé en acide libre, est présent dans une quantité comprise entre environ 1 mg/ml et environ 400 mg/ml, de préférence entre environ 1 mg/ml et 300 mg/ml, et plus préférentiellement entre 10 mg/ml et 50 mg/ml de la dite formulation.

8. La formulation d'acéclofénac selon l'une des revendications 1 et 5, où ledit composant solubilisant non aqueux est sélectionné dans le groupe composé du propylèneglycol, du polyéthylèneglycol et de l'éthanol facultatif.

9. La formulation d'acéclofénac selon l'une des revendications 1 et 5, où ledit composant solubilisant non aqueux est sélectionné dans le groupe composé du polyéthylèneglycol et de l'éthanol facultatif.

10. La formulation d'acéclofénac selon l'une des revendications 1 et 5, où ledit composant solubilisant non aqueux est sélectionné dans le groupe composé du propylèneglycol et du polyéthylèneglycol.

11. La formulation d'acéclofénac selon l'une des revendications 1 et 5, où de l'alcool benzylique est utilisé comme agent de conservation.

12. La formulation d'acéclofénac selon l'une des revendications 8 et 10, où la concentration dudit propylèneglycol est supérieure à 7 % en poids dudit liquide solvant de la revendication 1, lequel est formé par mélange dudit composant solubilisant non aqueux et dudit composant stabilisant de sel d'acéclofénac.

13. La formulation d'acéclofénac selon l'une des revendications 8 et 10, où la concentration dudit propylèneglycol est comprise entre environ 7 % et environ 70 % en poids dudit liquide solvant de la revendication 1, lequel est formé par mélange dudit composant solubilisant non aqueux et dudit composant stabilisant de sel d'acéclofénac.

14. La formulation d'acéclofénac selon l'une des revendications 8, 9 et 10, où la concentration dudit polyéthylèneglycol est comprise entre environ 35 % et environ 95 %, et de préférence supérieure à 30 %, en poids dudit liquide solvant de la revendication 1, lequel est formé par mélange dudit composant solubilisant non aqueux et dudit composant stabilisant de sel d'acéclofénac, et où ledit polyéthylèneglycol a une masse moléculaire moyenne comprise entre environ 200 et environ 1000, et de préférence entre environ 300 et environ 800.

15. La formulation d'acéclofénac selon la revendication 11, où la concentration dudit alcool benzylique est comprise entre environ 0,1 % et environ 4 % en poids dudit liquide solvant de la revendication 1.

16. La formulation d'acéclofénac selon la revendication 4, où la concentration desdits dérivés d'acide salicylique est comprise entre environ 1 % et environ 500 %, de préférence entre environ 50 % et environ 250 % et plus préférentiellement entre environ 75 % et environ 150 % en poids dudit sel d'acéclofénac de la revendication 1.

17. La formulation d'acéclofénac selon l'une des revendications 8 et 9, où une concentration dudit éthanol facultatif est comprise entre environ 1 % et environ 30 % en poids dudit liquide solvant de la revendication 1, lequel est formé par mélange dudit composant solubilisant non aqueux et dudit composant stabilisant de sel d'acéclofénac.

18. La formulation d'acéclofénac selon l'une des revendications 1, 3 et 4, où ledit composant stabilisant de sel d'acéclofénac comprend un moyen de limitation d'oxygène servant à limiter l'exposition effective de ladite formulation d'acéclofénac à l'oxygène, ledit moyen comprenant un ou plusieurs antioxydants sélectionnés dans le groupe composé de l'hydroxyanisol butylé, du gallate de propyle et de l'hydroxytoluène butylé, la quantité totale dudit antioxydant étant comprise entre environ 0,001 % et environ 5 % en poids dudit liquide solvant, et ledit moyen comprenant en outre une atmosphère limitée en gaz inerte en contact avec ladite formulation d'acéclofénac.

19. La formulation d'acéclofénac selon la revendication 1, où un dosage pharmaceutiquement efficace de celle-ci inclut ledit sel d'acéclofénac dans une concentration appropriée pour une administration parentérale sans autre dilution.

20. La formulation d'acéclofénac selon la revendication 19, où une quantité requise dudit dosage efficace est prévue dans un récipient scellé étanche à l'air qui est sélectionné dans le groupe composé d'une fiole, d'une ampoule, d'une seringue, d'un sachet, d'une poche et d'un injecteur automatique.

21. La formulation d'acéclofénac selon la revendication 20, où l'espace intérieur dudit récipient scellé étanche à l'air comprend un volume de remplissage occupé par ladite formulation d'acéclofénac et un volume d'expansion occupé par une microatmosphère limitée en gaz inerte, laquelle microatmosphère est constituée essentiellement d'un ou de plusieurs gaz inertes sélectionnés dans le groupe composé des gaz nobles et de l'azote de telle sorte que le rapport dudit volume de remplissage audit volume d'expansion ne soit pas inférieur à 1:1.

22. La formulation d'acéclofénac selon la revendication 21, où ledit gaz inerte d'azote dans ledit volume d'expansion a un volume d'azote ne dépassant pas 5 %, de manière à remplacer l'oxygène dans ledit volume d'expansion.

23. La formulation d'acéclofénac selon la revendication 19, où ledit sel d'acéclofénac est présent dans une quantité correspondant à une dose unitaire unique, ledit récipient scellé étanche à l'air de l'une des revendications 20 et 21 étant de préférence une ampoule en verre.

24. La formulation d'acéclofénac selon la revendication 19, où ledit sel d'acéclofénac est présent dans une quantité correspondant à un nombre de doses unitaires compris entre 1 (dose unique) et 20.

25. La formulation d'acéclofénac selon la revendication 19, où ledit sel d'acéclofénac est du sodium d'acéclofénac, rempli aseptiquement sous une enveloppe de gaz inerte.

26. Procédé d'élaboration d'une formulation parentérale d'acéclofénac liquide non aqueux convenant à une application pharmaceutique, lequel procédé comprend les étapes de :
(A) combinaison dans un ordre spécifique et mélange :
- (a) d'un composant acéclofénac sous forme de sel d'acéclofénac non soluble dans l'eau,
- (b) d'un composant solubilisant non aqueux permettant de stabiliser l'acéclofénac et le diclofénac qui se forme par conversion d'acéclofénac, ledit composant solubilisant non aqueux étant sensiblement inerte par rapport à cette conversion ;
- (c) d'un composant stabilisant de sel d'acéclofénac tel que des dérivés d'acide salicylique et un composant stabilisant de sel d'acéclofénac facultatif comportant des facteurs de limitation d'oxygène,
- (d) d'un composant éthanol facultatif, et
- (e) d'un composant alcool benzylique,
(B) solubilisation et/ou dissolution de sel d'acéclofénac dans le liquide solvant formé par mélange des composants de (b), (c), (d) et (e) ci-dessus de l'étape (A), où, en cas de stockage de ladite formulation parentérale d'acéclofénac liquide non aqueux dans un récipient fermé maintenu à 30 degrés Celsius pendant une durée de 180 jours, ledit acéclofénac constitue au moins environ 98 % en poids de la dite formulation d'acéclofénac, exprimée en acide libre d'acéclofénac dans ladite formulation.

27. Le procédé selon la revendication 26, où ledit composant stabilisant de sel d'acéclofénac de l'étape (c) de l'étape (A) comprend un ou plusieurs agents stabilisants sélectionnés dans le groupe composé des moyens de régulation du pH, des antioxydants, du propylèneglycol et du polyéthylèneglycol.

28. Le procédé selon la revendication 26, où au moins l'un dudit composant solubilisant non aqueux et dudit composant stabilisant de sel d'acéclofénac est du polyéthylèneglycol.

29. Le procédé selon la revendication 27, où la concentration dudit propylèneglycol est supérieure à 7 % en poids de ladite formulation.

30. Utilisation d'une formulation d'acéclofénac liquide non aqueux selon la revendication 1 pour l'élaboration d'une quantité administrable par voie parentérale et thérapeutiquement efficace pour traiter un sujet présentant un état ou un trouble pour lequel un traitement au NSAID est indiqué.

31. Utilisation d'une formulation d'acéclofénac liquide non aqueux selon la revendication 30, où ledit trouble est induit par du COX-2 et où ladite quantité administrable par voie parentérale et thérapeutiquement efficace correspond à une dose journalière d'acéclofénac liquide non aqueux comprise entre environ 1 mg par jour et environ 300 mg par jour, de préférence entre environ 10 mg par jour et environ 250 mg par jour, plus préférentiellement entre environ 30 mg par jour et environ 200 mg par jour, et encore plus préférentiellement entre environ 50 mg par jour et environ 150 mg par jour.
